# EUROPÄISCHE PATENTSCHRIFT

(11) **EP 2 453 288 B1**
(45) Veröffentlichungstag und Bekanntmachung des Hinweises auf die Patenterteilung: **04.05.2016**
(21) Anmeldenummer: 11187057.2
(22) Anmeldetag: 28.10.2011
(51) Int. Cl.: G02B 23/24, A61B 1/00

(54) **Objektiveinheit für Endoskope**
Objective unit for endoscopes
Unité d'objectif pour endoscopes

(30) Priorität: 11.11.2010 DE 102010050932
(43) Veröffentlichungstag der Anmeldung: 16.05.2012
(73) Patentinhaber: Karl Storz GmbH & Co. KG, 78532 Tuttlingen (DE)
(72) Erfinder: Dahmen, Jan, 78532 Tuttlingen (DE)

(56) Entgegenhaltungen:
- DE-A1- 19 806 984
- DE-B3-102006 017 683
- US-A- 5 512 036
- US-A- 5 609 561
- US-A- 5 961 445
- US-A1- 2008 027 276

## Beschreibung

Die Erfindung betrifft eine Objektiveinheit für Endoskope, mit einem mindestens eine Linse aufweisenden Objektiv sowie mit einer Objektivfassung, die so in einem Endoskopgehäuse gelagert ist.

Derartige Objektiveinheiten sind aus der Praxis in verschiedenen Ausgestaltungsformen bekannt. Um einerseits das Objektiv in Längsrichtung des optischen Systems zu justieren und andererseits die mit dem Objektiv bestückte Objektivfassung lagesicher im Endoskopgehäuse zu fixieren, ist es aus der Praxis bekannt, die Objektivfassung über ein Andrückelement im Endoskopgehäuse zu positionieren, das seinerseits im montierten Zustand mit dem Endoskopgehäuse unlösbar verkittet wird.

Diese bekannten Objektiveinheiten haben sich in der Praxis durchaus bewährt, jedoch besteht einerseits die Gefahr von Verunreinigungen durch das Verkitten der Bauteile und andererseits besteht keine Möglichkeit, eine eventuelle Fehlstellung des Objektivs bezüglich der optischen Achse nachträglich zu korrigieren, da die Objektiveinheit nicht mehr demontierbar ist.

Das Patent US 5,961,445 schlägt eine Objektiveinheit für Endoskope nach dem Oberbegriff von Anspruch 1 vor, die ein Objektiv mit mindestens einer Linse aufweist, sowie eine in einem Endoskopgehäuse lagerbare Objektivfassung, die über eine Rastverbindung lösbar im Endoskopgehäuse festlegbar ist, wobei ein proximal angeordnetes Bauteil als Rastelement wirkt.

Davon ausgehend liegt der Erfindung die **Aufgabe** zugrunde, eine Objektiveinheit für Endoskope zu schaffen, die bei einfacher Montage positionsstabil im Endoskopgehäuse positionierbar ist.

Die Lösung dieser Aufgabenstellung ist erfindungsgemäß durch die Merkmale von Auspruch 1 definiert und dadurch gekennzeichnet, dass das proximale Ende der Objektivfassung federelastisch dazu ausgebildet ist, dass sich die Objektivfassung, beim Einschieben in das Endoskopgehäuse, radial nach innen zusammendrücken lässt, und das Rastelement beim Erreichen einer entsprechenden Position aufgrund der Expansion der Objektivfassung in radialer Richtung automatisch in eine im Endoskopgehäuse ausgebildete Rastaufnahme eingreift.

Durch die erfindungsgemäße Objektiveinheit ist es möglich, die Objektiveinheit nachträglich zu demontieren und das Objektiv bei einer möglichen Fehlstellung erneut positionsgenau auszurichten.

Die hierin gezeigten Rastverbindungen zeichnen sich dadurch aus, dass sie bei einfacher Handhabung einen sicheren Halt der miteinander verrasteten Bauteile gewährleisten, jedoch auch einfach und schnell wieder zu trennen sind.

Um den Druck der Objektiveinheit in axialer Richtung, das heißt insbesondere in Richtung auf das Deckglas zu verhindern, bzw. zu begrenzen, wobei der Druck über die Objektivfassung auf das mit der Objektivfassung verbundene Objektiv übertragen wird, wird gemäß einer ersten, nicht zu Erfindung gelörenden Ausführungsform vorgeschlagen, dass die Einschubtiefe der Objektiveinheit in das Endoskopgehäuse in distaler Richtung über einen Anschlag für die Objektivfassung begrenzbar ist.

Gemäß einer Ausführungsform ist der Anschlag als Absatz im Endoskopgehäuse ausgebildet ist, gegen den die Objektivfassung in der distalen Endposition anläuft.

Mit einer alternativen Ausführungsform wird vorgeschlagen, dass der Anschlag als Rastverbindung ausgebildet ist, über die die Objektivfassung lösbar im Endoskopgehäuse festlegbar ist. Bei dieser praktischen Ausführungsform werden durch die Rastverbindung gleich zwei Vorteile erzielt, nämlich die lösbare Lagerung der Objektivfassung im Endoskopgehäuse und die Begrenzung der Einschubtiefe der Objektiveinheit in das Endoskopgehäuse.

Mit einer ersten Ausführungsform zur Ausbildung eines erfindungsgemäßen Endoskops mit Objektiveinheit gemäß Anspruch 3 wird vorgeschlagen, dass die Rastverbindung aus mindestens einem an der Objektivfassung angeordneten Rastelement und mindestens einer im Endoskopgehäuse ausgebildeten Rastaufnahme zur Aufnahme jeweils eines Rastelements besteht.

Dabei ist das Rastelement am federelastisch ausgebildeten proximalen Ende der Objektivfassung angeordnet. Die Federelastizität des proximalen Endes der Objektivfassung sorgt dafür, dass das Rastelement automatisch in die zugehörige Rastaufnahme eingreift, sobald die beiden Bauteile zueinander in Deckung gebracht werden.

Alternativ jedoch nicht zum Gegested der Erfindung gelöred, kann auch das beispielsweise als Stift oder Kugel ausgebildete mindestens eine Rastelement im Endoskopgehäuse und die Rastaufnahme an der Objektivfassung angeordnet sein.

Weiterhin wird für die erste Ausführungsform der Erfindung vorgeschlagen, dass die Rastverbindung über ein von proximal in die Objektivfassung einsetzbares Halteelement in der Rastposition fixierbar ist, wobei das mindestens eine Rastelement über das Halteelement radial nach außen in die entsprechende Rastaufnahme gedrückt wird. Durch die radiale Andrückkraft des Halteelements wird das Rastelement lagesicher in der Rastaufnahme fixiert.

Zum Lösen der Rastverbindung wird als weitere Ausführungsform der Erfindung vorgeschlagen, dass das federelastische proximale Ende der Objektivfassung mittels eines von proximal in das Endoskopgehäuse einsetzbaren Demontagewerkzeugs radial nach innen biegbar ist. Bei der Verwendung des Halteelements zum Fixieren der Rastverbindung muss dieses selbstverständlich zuvor aus der Objektivfassung entfernt werden.

Zum Lösen der nicht erfindungsgemäßen Rastverbindung, bei der das Rastelement im Endoskopgehäuse und die Rastaufnahme an der Objektivfassung angeordnet sind, wird vorgeschlagen, dass das Rastelement mittels eines von proximal an die Objektivfassung ansetzbaren Demontagewerkzeugs radial nach außen in das Endoskopgehäuse drückbar ist.

Mit einer dritten nicht zur Erfindung gelörende Ausführungsform zur Ausbildung der lösbaren Rastverbindung wird schließlich vorgeschlagen, dass die Objektivfassung über eine Andrückeinheit lösbar im Endoskopgehäuse festlegbar ist, die die Objektivfassung in distaler Richtung belastet, wobei die Andrückeinheit vorteilhafterweise aus einem als Druckfeder ausgebildeten Federelement sowie einem am Endoskopgehäuse festlegbaren Widerlager für die Druckfeder besteht und das Widerlager über mindestens eine Rastverbindung lösbar am Endoskopgehäuse festlegbar ist. Die Verwendung der Druckfeder in der Andrückeinheit dient dazu, die Objektivfassung in distaler Richtung gegen den Absatz im Endoskopgehäuse zu drücken und so das mit der Objektivfassung verbundene Objektiv in axialer Richtung positionsstabil im Endoskopgehäuse zu positionieren

Zur Ausbildung des Widerlagers wird vorgeschlagen, dass dieses als geschlitzte federelastische Hülse ausgebildet ist, wobei der distalseitige Rand der Hülse einen umlaufenden Rastvorsprung aufweist, der im montierten Zustand zur Ausbildung der lösbaren Rastverbindung in eine umlaufende Rastnut im Endoskopgehäuse eingreift. Der Widerlagerabschnitt, an dem das Federelement unmittelbar anliegt ist der proximale Abschnitt der Hülse, der als Ringabschnitt ausgebildet ist.

Schließlich wird vorgeschlagen, dass die Hülse sich in proximaler Richtung konisch verjüngend ausgebildet ist, so dass zum Lösen der Rastverbindung zwischen Hülse und Endoskopgehäuse ein Demontagerohr auf die konische Außenwand der Hülse aufschiebbar ist, wodurch die federelastische Hülse von ihrem proximalen Ringabschnitt radial nach innen zusammengedrückt wird, bis der umlaufende Rastvorsprung außer Eingriff mit der im Endoskopgehäuse ausgebildeten Rastnut tritt.

Weitere Merkmale und Vorteile der Erfindung ergeben sich teils anhand der zugehörigen Zeichnungen, in denen Ausführungsbeispiele einer erfindungsgemäßen Objektiveinheit für Endoskope nur beispielhaft dargestellt sind, ohne die Erfindung auf diese Ausführungsbeispiele zu beschränken. In den Zeichnungen zeigt:
- Fig. 1: einen Längsschnitt durch eine erste Ausführungsform einer nicht erfindungsgemäßen Objektiveinheit;
- Fig.2: einen Längsschnitt durch eine zweite und dritte erfindungsgemäße Ausführungsform einer Objektiveinheit;
- Fig. 3: einen Längsschnitt durch eine vierte Ausführungsform einer erfindungsgemäßen Objektiveinheit und
- Fig. 4: einen Längsschnitt durch eine fünfte, nicht erfindungsgemäße Ausführungsform einer weiteren Objektiveinheit.

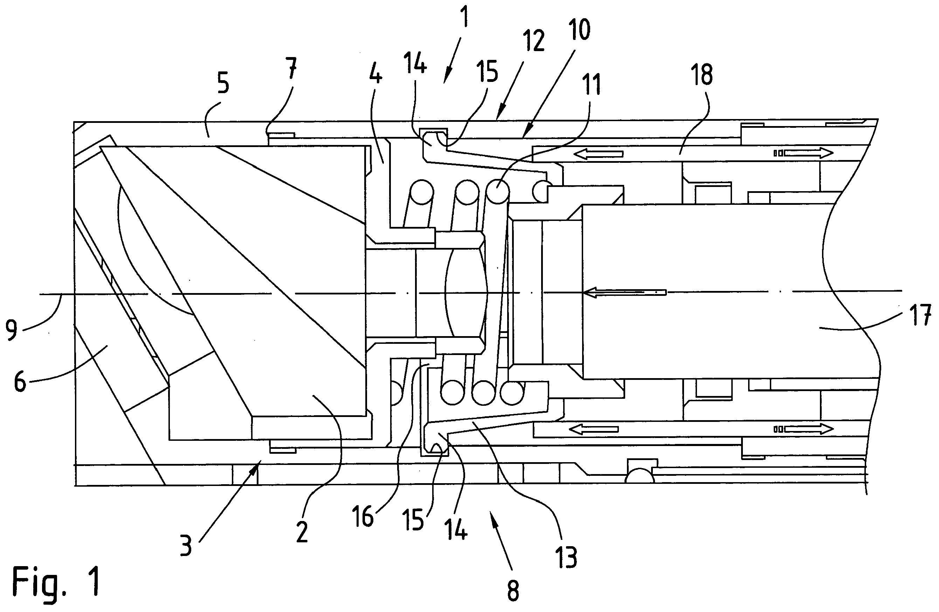

Die in den Abbildungen Fig. 1 bis Fig. 4 dargestellten Objektiveinheiten 1 für Endoskope bestehen im Wesentlichen aus einem mindestens eine Linse 2 aufweisenden Objektiv 3 sowie einer Objektivfassung 4, wobei die Objektiveinheit 1 so in einem Endoskopgehäuse 5 gelagert ist, dass die Objektiveinheit 1 distalseitig mit einem Luftabstand zu einem im Endoskopgehäuse 5 festgelegten Deckglas 6 angeordnet ist und die im Endoskopgehäuse 5 festlegbaren Objektivfassung 4 das proximalseitige Ende der Objektiveinheit 1 bildet.

Um die Einbautiefe der Objektiveinheit 1 in das Endoskopgehäuse 5 in distaler Richtung zu begrenzen, ist bei den in Fig. 1 bis 3 dargestellten Ausführungsformen im Endoskopgehäuse 5 ein als Absatz 7 ausgestalteter Anschlag ausgebildet, gegen den die, beispielsweise durch Verkleben, mit dem Objektiv 3 verbundene Objektivfassung 4 beim Einschieben in das Endoskopgehäuse 5 anläuft und so einerseits ein weiteres Einschieben in distaler Richtung verhindert, wodurch eine Druckbelastung auf die optischen Bauteile der Objektiveinheit 1 verhindert wird, und andererseits eine positionsgenaue Montage des Objektives 3 gewährleistet wird.

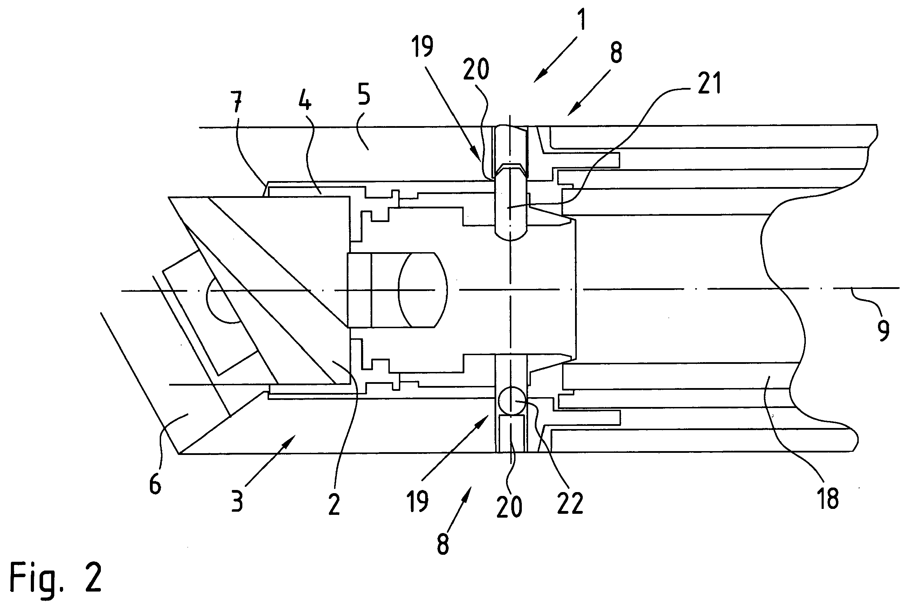

Alternativ und/oder zusätzlich wird die Einbautiefe der Objektiveinheit 1 in distaler Richtung in das Endoskopgehäuse 5 bei den in Fig. 2 bis 4 dargestellten Ausführungsformen durch eine Rastverbindung 8 begrenzt, über die die das Objektiv 3 proximalseitig führende Objektivfassung 4 lösbar im Endoskopgehäuse 5 festlegbar ist. Sobald die Rastverbindung 8 beim Einschieben der Objektivfassung 4 in das Endoskopgehäuse 5 verrastet und somit die Objektivfassung 4 und das Endoskopgehäuse 5 relativ zueinander fixiert, kann auch das mit der Objektivfassung 4 verbundene Objektiv 3 nicht weiter in distaler Richtung in das Endoskopgehäuse 5 eingeschoben werden.

Die Verwendung der Rastverbindung 8 zum Festlegen der Objektivfassung 4 im Endoskopgehäuse 5 hat darüber hinaus den Vorteil, dass die Objektiveinheit 1 leicht zu montieren und wieder zu demontieren ist. Gerade die nachträgliche Demontierbarkeit der Objektiveinheit 1 stellt einen wesentlichen Vorteil gegenüber dem Stand der Technik dar, da beispielsweise Fehlausrichtungen des Objektivs 3 bezüglich der optischen Achse 9 korrigierbar sind und Verunreinigungen, wie sie beim Verkitten der herkömmlichen Objektiveinheiten 1 auftreten können, vollkommen ausgeschlossen sind.

Die in den Abbildungen Fig. 1 bis Fig. 4 dargestellten Ausführungsbeispiele unterscheiden sich durch die Ausbildung der Objektivfasssungen 4 sowie der Rastverbindungen 8 zum lösbaren Lagern der Objektivfassungen 4 in den Endoskopgehäusen 5 voneinander.

Bei der in Fig. 1 dargestellten ersten Ausführungsform ist die Objektivfassung 4 über eine Andrückeinheit 10 lösbar im Endoskopgehäuse 5 festlegbar, die die Objektivfassung 4 und somit auch das mit der Objektivfassung 4 verbundene Objektiv 3 in distaler Richtung drückt, wobei die Andrückeinheit 10 aus einem als Druckfeder 11 ausgebildeten Federelement sowie einem mittels der Rastverbindung 8 am Endoskopgehäuse 5 festlegbaren Widerlager 12 für die Druckfeder 11 besteht.

Die Verwendung der Druckfeder 11 in der Andrückeinheit 10 dient dazu, die Objektivfassung 4 in distaler Richtung gegen den Absatz 7 im Endoskopgehäuse 5 zu drücken und so das mit der Objektivfassung 4 verbundene Objektiv 3 in axialer Richtung positionsstabil im Endoskopgehäuse 5 zu positionieren.

Wie weiterhin aus Fig. 1 ersichtlich, ist das Widerlager 12 als geschlitzte federelastische Hülse 13 ausgebildet, wobei der distalseitige Rand der Hülse 13 einen umlaufenden Rastvorsprung 14 aufweist, der im montierten Zustand in eine umlaufende Rastnut 15 im Endoskopgehäuse 5 eingreift, um die lösbare Rastverbindung 8 zu bilden. Proximalseitig ist die Hülse 13 sich konisch verjüngend ausgebildet. Die Federelastizität der Hülse 13 wird durch Schlitze 16 erzielt, die vom distalen Ende der Hülse 13 aus in axialer Richtung der Hülse 13 ausgebildet sind.

Die Federelastizität der Hülse 13 dient dazu, dass sich die Hülse 13 beim Einschieben in das Endoskopgehäuse 5 radial nach innen zusammendrücken lässt und der umlaufende Rastvorsprung 14 beim Erreichen der entsprechenden Position aufgrund der Expansion der Hülse 13 in radialer Richtung automatisch in die im Endoskopgehäuse 5 ausgebildete Rastnut 15 eingreift, um das Widerlager 12 und somit auch die Objektivfassung 4 bzw. die gesamte Objektiveinheit 1 in axialer Richtung im Endoskopgehäuse 5 zu fixieren.

Wie in Fig. 1 dargestellt, kann zum Einsetzen der Objektiveinheit 1 in das Endoskopgehäuse 5 ein Montagedorn 17 verwendet werden, der von proximal an die Hülse 13 ansetzbar ist.

Zum Lösen der Rastverbindung 8 und somit zur Demontage der Objektiveinheit 1 ist zwischen die Hülse 13 und das Endoskopgehäuse 5 ein, beispielsweise als Rohr ausgebildetes, Demontagewerkzeug 18 auf die konische Außenwand der Hülse 13 aufschiebbar, wodurch die federelastisch ausgebildete Hülse 13 radial nach innen zusammengedrückt wird, bis der umlaufende Rastvorsprung 14 außer Eingriff mit der im Endoskopgehäuse 5 ausgebildeten Rastnut 15 tritt.

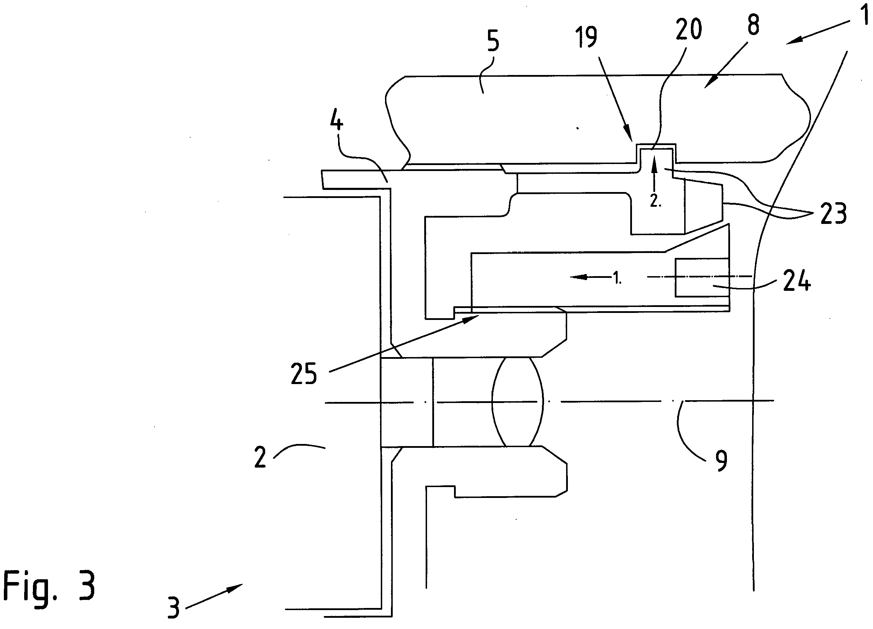

Bei den in Fig. 2 und 3 dargestellten Ausführungsformen besteht die Rastverbindung 8 aus mindestens einem an der Objektivfassung 4 angeordneten Rastelement 19 und mindestens einer im Endoskopgehäuse 5 ausgebildeten Rastaufnahme 20 zur Aufnahme jeweils eines Rastelements 19, wobei das mindestens eine Rastelement 19 am federelastisch ausgebildeten proximalen Ende der Objektivfassung 4 angeordnet ist. Dabei ist die Rastaufnahme 20 in Form einer Bohrung ausgestaltet.

Die federelastische Ausbildung des proximalen Endes der Objektivfassung 4 dient dazu, dass sich die Objektivfassung 4 beim Einschieben in das Endoskopgehäuse 5 radial nach innen zusammendrücken lässt und das mindestens eine Rastelement 19 beim Erreichen der entsprechenden Position aufgrund der Expansion der Objektivfassung 4 in radialer Richtung automatisch in die im Endoskopgehäuse 5 ausgebildete Rastaufnahme 20 eingreift, um die Objektivfassung 4 bzw. die gesamte Objektiveinheit 1 in axialer Richtung im Endoskopgehäuse 5 spielfrei zu fixieren. Ebenso ist eine Dreh-Fixierung gewährleistet, da die Rastaufnahme 20 bzw. Rastaufnahmen 20 an konkreten Umfangspositionen des Endoskopgehäuses 5 ausgebildet ist bzw. sind.

Wie aus Fig. 2 ersichtlich, ist zum Lösen der Rastverbindung 8 und somit zur Demontage der Objektiveinheit 1 auf das federelastische proximale Ende der Objektivfassung 4 ein, beispielsweise als Rohr ausgebildetes, Demontagewerkzeug 18 aufschiebbar, wodurch das federelastisch ausgebildete proximale Ende der Objektivfassung 4 radial nach innen zusammengedrückt wird, bis das mindestens eine Rastelement 19 außer Eingriff mit der im Endoskopgehäuse 5 ausgebildeten Rastaufnahme 20 tritt.

Zur Ausbildung des am federelastischen proximalen Ende der Objektivfassung 4 angeordneten mindestens einen Rastelements 19 sind in Fig. 2 zwei Ausführungsbeispiele dargestellt, nämlich die Ausbildung des Rastelements 19 als Stift 21 - im oberen Teil der Abbildung Fig. 2 - oder als Kugel 22 - im unteren Teil der Abbildung Fig. 2.

Bei der in Fig. 3 dargestellten vierten Ausführungsform ist das mindestens eine Rastelement 19 der Rastverbindung 8 als am federelastischen Ende der Objektivfassung 4 angeformte Rastnase 23 ausgebildet, die in die entsprechende Rastaufnahme 20 im Endoskopgehäuse 5 rastend eingreift. Bei dieser Ausführungsform ist die Rastaufnahme 20 bevorzugt als ringförmige Rastaufnahme, beispielsweise Ringnut 20, ausgebildet.

Darüber hinaus zeigt die Abbildung Fig. 3, dass die Rastverbindung 8 über ein von proximal in die Objektivfassung 4 einsetzbares Halteelement 24 in der Rastposition insbesondere dreh-fixierbar ist. Bei der dargestellten Ausführungsform ist das Halteelement 24 über eine Gewindeverbindung 25 an der Objektivfassung 4 festlegbar.

Das Halteelement 24 ist im Wesentlichen rohrförmig und erweitert sich konisch nach proximal. Alternativ oder zusätzlich kann auch die dem Halteelement zugewandte Seite der Objektivfassung 4 im Bereich der Rastnase 23 konisch ausgebildet sein.

Durch die radiale Andrückkraft des Halteelements 24 wird das Rastelement 19 lagesicher und kraftschlüssig in der Rastaufnahme 20 fixiert. Hierdurch kann somit eine Dreh-Fixierung bewirkt werden, in diesem Fall eine kraftschlüssige Verbindung - statt einer formschlüssigen wie in der vorigen Ausführungsform.

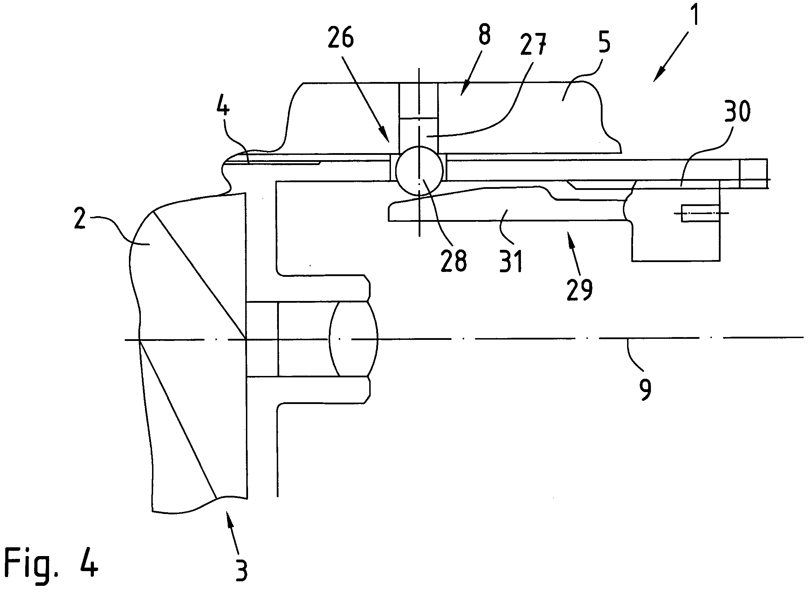

Bei der in Fig. 4 dargestellten fünften Ausführungsformen besteht die Rastverbindung 8 aus mindestens einem in der Objektivfassung 4 angeordneten Rastelement 26 und mindestens einer im Endoskopgehäuse 5 ausgebildeten Rastaufnahme 27 zur Aufnahme jeweils eines Rastelements 26, wobei das Rastelement 26 bei der dargestellten Ausführungsform beispielhaft als Kugel 28 ausgebildet ist, jedoch sind auch andere Ausgestaltungsformen möglich. Die Rastaufnahme 27 kann wie bei den vorherigen Ausführungsformen ringnutförmig ausgebildet sein oder auch als Bohrung bzw. Bohrungen an einer bzw. mehreren konkreten Umfangspositionen des Endoskopgehäuses 5.

Darüber hinaus zeigt die Abbildung Fig. 4, dass die Rastverbindung 8 über ein von proximal in die Objektivfassung 4 einsetzbares Halteelement 29 in die Rastposition überführbar und in dieser Position fixierbar ist, wobei die Kugel 28 über das Halteelement 29 radial nach außen in die entsprechende Rastaufnahme 27 gedrückt wird. Bei der dargestellten Ausführungsform ist das Halteelement 29 über eine Gewindeverbindung 30 an der Objektivfassung 4 festlegbar. Durch die radiale Andrückkraft des Halteelements 29 wird die Kugel 28 lagesicher und kraftschlüssig in der Rastaufnahme 27 fixiert. Hierdurch kann neben der axialen Sicherung die Objektivfassung 4 im Endoskopgehäuse 5 auch drehgesichert sein. Zum Einen, indem die Rastaufnahme 27 in Form der Ringnut ausgebildet ist und die Verbindung zwischen dem Rastelement 26 und der Rastaufnahme 27 bei für die Rastposition eingeschraubtem Halteelement 29 ausreichend kraftschlüssig ist. Zum anderen, indem die Rastaufnahme 27 in Form der konkreten Umfangsposition bzw. Umfangspositionen des Endoskopgehäuses 5 ausgebildet ist.

Das Eindrücken der Kugel 28 in die Rastaufnahme 27 im Endoskopgehäuse 5 erfolgt bei der dargestellten Ausführungsform des Halteelements 29 über einen federelastischen Andrückarm 31. Je nach Einschraubtiefe des Halteelements 29 in die Objektivfassung 4, wird die auf die Kugel 28 wirkende Federkraft des Andrückarms 31 erhöht oder reduziert. Dies wird erreicht, indem der Andrückarm 31 als Segment einer sich nach proximal erweiternden Hülse ausgeführt ist.

Zum Lösen der Rastverbindung 8 und somit zur Demontage der Objektiveinheit 1 wird das Halteelement 29 aus der Objektivfassung 4 herausgeschraubt, wodurch die Kugel 28 radial nach innen aus der Rastaufnahme 27 des Endoskopgehäuses 5 fällt und die Rastverbindung 8 aufhebt. Die Objektiveinheit 1 kann nachfolgend nach proximal aus dem Endoskopgehäuse 5 gezogen werden.

Die wie voranstehend beschrieben ausgebildeten Objektiveinheiten 1 zeichnen sich dadurch aus, dass sie lösbar und somit demontierbar im Endoskopgehäuse 5 festlegbar sind.

**Bezugszeichenliste**

| | | | |
|---|---|---|---|
| 1 | Objektiveinheit | 18 | Demontagewerkzeug |
| 2 | Linse | 19 | Rastelement |
| 3 | Objektiv | 20 | Rastaufnahme |
| 4 | Objektivfassung | 21 | Stift |
| 5 | Endoskopgehäuse | 22 | Kugel |
| 6 | Deckglas | 23 | Rastnase |
| 7 | Absatz | 24 | Halteelement |
| 8 | Rastverbindung | 25 | Gewindeverbindung |
| 9 | optische Achse | 26 | Rastelement |
| 10 | Andrückeinheit | 27 | Rastaufnahme |
| 11 | Druckfeder | 28 | Kugel |
| 12 | Widerlager | 29 | Halteelement |
| 13 | Hülse | 30 | Gewindeverbindung |
| 14 | Rastvorsprung | 31 | Andrückarm |
| 15 | Rastnut | | |
| 16 | Schlitz | | |
| 17 | Montagedorn | | |

## Patentansprüche

1. Objektiveinheit für Endoskope mit einem Endoskopgehäuse (5) und einem im Endoskopgehäuse festgelegten Deckglas (6), mit einem mindestens eine Linse (2) aufweisenden Objektiv (3) und mit einer Objektivfassung (4), wobei die Objektivfassung (4) das proximalseitige Ende der Objektiveinheit (1) bildet,
wobei an einem proximalen Ende der Objektivfassung (4) mindestens ein Rastelement (19;21;22;23) angeordnet ist,
**dadurch gekennzeichnet, dass**
das proximale Ende der Objektivfassung (4) federelastisch dazu ausgebildet ist, dass sich die Objektivfassung (4), vorzugsweise beim Einschieben in das Endoskopgehäuse (5), radial nach innen zusammendrücken lässt, und
das mindestens eine Rastelement (19; 21; 22; 23) geeignet ist, aufgrund der Expansion der Objektivfassung (4) in radialer Richtung in eine im Endoskopgehäuse (5) ausgebildete Rastaufnahme (20) einzugreifen.

2. Objektiveinheit nach Anspruch 1, **dadurch gekennzeichnet, dass** das mindestens eine Rastelement (19; 21; 22; 23) als Stift (21) oder Kugel (22) ausgebildet ist.

3. Endoskop mit einer Objektiveinheit nach Anspruch 1 oder 2 und mit einem Endoskopgehäuse (5) mit einem darin festgelegten Deckglas (6) und mit mindestens einer darin ausgebildeten Rastaufnahme (20) zur Aufnahme des Rastelements (19; 21; 22; 23) der Objektivfassung (4),
wobei die Objektivfassung (4) über eine aus dem Rastelement (19; 21; 22; 23) und der Rastaufnahme (20) bestehende Rastverbindung (8) lösbar im Endoskopgehäuse (5) festlegbar ist,
wobei das federelastisch ausgebildete proximale Ende der Objektivfassung (4) so ausgebildet ist, dass sich die Objektivfassung (4) beim Einschieben in das Endoskopgehäuse (5) radial nach innen zusammendrücken lässt, und das mindestens eine Rastelement (19; 21; 22; 23) beim Erreichen einer entsprechenden Rastposition automatisch in die Rastaufnahme (20) eingreift,
**gekennzeichnet durch** ein von proximal in die Objektivfassung (4) einsetzbares Halteelement (24), über das die Rastverbindung (8) in der Rastposition fixierbar ist, wobei das Halteelement (24) das mindestens eine Rastelement (19; 21; 22; 23) radial in die entsprechende Rastaufnahme (20) drückt.

## Claims

1. Objective unit for endoscopes, with an endoscope housing (5) and a cover glass (6) secured in the endoscope housing (5), with an objective (3) having at least one lens (2), and with an objective mount (4), wherein the objective mount (4) forms the proximal-side end of the objective unit (1),
wherein at least one catch element (19; 21; 22; 23) is arranged on a proximal end of the objective mount (4),
**characterized in that**
the proximal end of the objective mount (4) is designed to be resiliently elastic, such that the objective mount (4), preferably upon insertion into the endoscope housing (5), can be compressed radially inwards, and
the at least one catch element (19; 21; 22; 23) is suitable, on account of the expansion of the objective mount (4) in the radial direction, for engaging in a catch seat (20) formed in the endoscope housing (5).

2. Objective unit according to Claim 1, **characterized in that** the at least one catch element (19; 21; 22; 23) is designed as a pin (21) or ball (22).

3. Endoscope with an objective unit according to Claim 1 or 2 and with an endoscope housing (5) with a cover glass (6) secured therein and with at least one catch seat (20) formed therein for the purpose of receiving the catch element (19; 21; 22; 23) of the objective mount (4),
wherein the objective mount (4) can be secured releasably in the endoscope housing (5) by a catch connection (8) composed of the catch element (19; 21; 22; 23) and of the catch seat (20),
wherein the resiliently elastic proximal end of the objective mount (4) is designed such that the objective mount (4) can be compressed radially inwards upon insertion into the endoscope housing (5), and the at least one catch element (19; 21; 22; 23), on reaching a corresponding catch position, engages automatically in the catch seat (20),
**characterized by** a holding element (24), which can be inserted from the proximal direction into the objective mount (4) and by which the catch connection (8) can be fixed in the catch position, wherein the holding element (24) presses the at least one catch element (19; 21; 22; 23) radially into the corresponding catch seat (20).

## Revendications

1. Unité d'objectif pour endoscope comportant un boîtier d'endoscope (5) et un verre de protection (6) fixé dans l'endoscope, comportant au moins un objectif (3) comprenant des lentilles (2) et comportant une monture d'objectif (4), dans laquelle la monture d'objectif (4) forme l'extrémité du côté proximal de l'unité d'objectif (1),
dans laquelle au moins un élément d'encliquetage (19 ; 21 ; 22 ; 23) est disposé sur une extrémité proximale de la monture d'objectif (4),
**caractérisé en ce que**
l'extrémité proximale de la monture d'objectif (4) est réalisée de manière à présenter l'élasticité d'un ressort afin que la monture d'objectif (4) puisse être comprimée radialement vers l'intérieur, de préférence lors de son insertion dans le boîtier d'endoscope (5), et
l'au moins un élément d'encliquetage (19 ; 21 ; 22 ; 23) est approprié pour s'engager dans un réceptacle d'encliquetage (20) formé dans le boîtier d'endoscope (5) du fait de la dilatation de la monture d'objectif (4) en direction radiale.

2. Unité d'objectif selon la revendication 1, **caractérisée en ce que** l'au moins un élément d'encliquetage (19 ; 21 ; 22 ; 23) est réalisé sous la forme d'une tige (21) ou d'une sphère (22).

3. Endoscope comportant une unité d'objectif selon la revendication 1 ou 2 et comportant un boîtier d'endoscope (5) muni d'un verre de protection (6) fixé dans celui-ci et comportant au moins un réceptacle d'encliquetage (20) formé dans celui-ci pour recevoir l'élément d'encliquetage (19 ; 21 ; 22 ; 23) de la monture d'objectif (4),
dans lequel la monture d'objectif (4) peut être fixée de manière amovible dans le boîtier d'endoscope (5) par l'intermédiaire d'une liaison d'encliquetage (8) constituée de l'élément d'encliquetage (19 ; 21 ; 22 ; 23) et du réceptacle d'encliquetage (20),
dans lequel l'extrémité proximale réalisée de manière à présenter l'élasticité d'un ressort de la monture d'objectif (4) est réalisée de manière à ce que la monture d'objectif (4) puisse être comprimée radialement vers l'intérieur lors de son insertion dans le boîtier d'endoscope (5), et l'au moins un élément d'encliquetage (19 ; 21 ; 22 ; 23) s'engage automatiquement dans le réceptacle d'encliquetage (20) lorsqu'une position d'encliquetage correspondante a été atteinte,
**caractérisé par** un élément de maintien (24) pouvant être inséré de manière proximale dans la monture d'objectif (4), par l'intermédiaire duquel la liaison d'encliquetage (8) peut être fixée à la position d'encliquetage, dans lequel l'élément de maintien (24) comprime radialement l'au moins un élément d'encliquetage (19 ; 21 ; 22 ; 23) dans le réceptacle d'encliquetage (20) correspondant.
